# EUROPÄISCHE PATENTANMELDUNG

(11) **EP 1 279 741 A1**
(43) Veröffentlichungstag der Anmeldung: **29.01.2003**
(21) Anmeldenummer: 02014064.6
(22) Anmeldetag: 01.07.2002
(51) Int. Cl.: C12N 15/90, C12N 15/66

(54) **Verfahren zum Herstellen von Deletionsmutanten**

(30) Priorität: 12.07.2001 DE 10133928
(71) Anmelder: Bayer CropScience AG, 40789 Monheim (DE)
(72) Erfinder: Kämper, Jörg, Dr., 35287 Amönenburg (DE); Schreier, Peter, Prof. Dr., 50674 Köln (DE)

(57) **Zusammenfassung**

Die vorliegende Erfindung betrifft ein neues Verfahren zum Herstellen von Deletionsmutanten, das auf einer PCR-Strategie basiert, eine universell einsetzbare Knockout-Kassette, die ein selektierbares Marker-Gen enthält und durch das spezifische Design der Restriktionsschnittstellen charakterisiert ist, sowie ein Verfahren zum Herstellen dieser Knockout-Kassette.

SEQUENZPROTOKOLL

<110> Bayer AG

<120> Verfahren zum Herstellen von Deletionsmutanten

<130> Le A 35 242

<160> 16

<170> PatentIn version 3.0

<210> 1
<211> 24
<212> DNA
<213> Primer lb1

<400> 1
gaagctagcg gaaaagtggg tgag 24

<210> 2
<211> 41
<212> DNA
<213> Primer lb2

<400> 2
cacggcctga gtggccgact ttgaaagcgc ccactggatg g 41

<210> 3
<211> 23
<212> DNA
<213> Primer rb1

<400> 3
ccgtagttgt gcgagagcag agg 23

<210> 4

<211> 40

<212> DNA

<213> Primer rb2

<400> 4
gtgggccatc taggccgatt tcagtgatac gtttagtccc 40

<210> 5

<211> 21

<212> DNA

<213> Primer KK-lb

<400> 5
actagatccg atgataagct g 21

<210> 6

<211> 20

<212> DNA

<213> Primer KK-rb

<400> 6
aactcgctgg tagttaccac 20

<210> 7

<211> 22

<212> DNA

<213> Primer test-lb

<400> 7
gtggaagatg ctggaccgaa gc 22

<210> 8

<211> 22

<212> DNA

<213>  Primer test-rb

<400>  8
accgtgcaac tcatcttcgg tg                                                22

<210>  9

<211>  13

<212>  DNA

<213>  SfiI-a (pBS-hhn)

<400>  9
ggcctagatg gcc                                                          13

<210>  10

<211>  13

<212>  DNA

<213>  SfiI-b (pBS-hhn)

<400>  10
ggcctgagtg gcc                                                          13

<210>  11

<211>  25

<212>  DNA

<213>  Primer hph-Nco/Bam

<400>  11
gcggatccca tggaaaagcc tgaac                                             25

<210>  12

<211>  32

<212>  DNA

<213>  Primer hph-stop

<400>  12
gggcggccgc tctattcctt tgccctcgga cg                                     32

<210> 13

<211> 44

<212> DNA

<213> Primer 3-hph

<400> 13
cggagctcgg ccactcaggc ctcatgtttg acagcttatc atcg 44

<210> 14

<211> 45

<212> DNA

<213> Primer 5-hsp

<400> 14
cgctcgaggc ctagatggcc gaacgtggta actaccagcg agttc 45

<210> 15

<211> 35

<212> DNA

<213> Primer hsp-Nco

<400> 15
cggaagcttc catggccccc cgggatctgg caacg 35

<210> 16

<211> 1908

<212> DNA

<213> XhoI/SacI-Insert (pBS-hhn)

<400> 16
ctcgaggcct agatggccga acgtggtaac taccagcgag ttctgcaaac ttcaaaaaaa 60

aaatctgggc acgatgaaag ttgagctaac gctgacgctc acaaatggcg tggctaaagg 120

aagcgagaca atcggaaaat tgttctctcg ggcaccacaa agctgttgtt agtcgctgaa 180

gaacaattcc aactgattcc gccgccttcc tattgcgtca gccttgtacc taagctgccg 240

agtaacgtca ctcaacctct cttttcagac tgctttgctc cgcgaatact tttcttctat 300

gcgctcaaga aaatgacaca gcacaccaag ctctgcaaac tttcttcgct aatctgacgc 360

gaaatgtgag ccatttcttc tcgcctgcaa tggcaatgcg tctgtgcggc gatgagaatc 420
acgatgcgga atgggtggct ggaagttcat agagatgctg agttgttgga gcgacatggt 480
acataagcat gagtctgtcc tgatttccac cctcccgtct ttcatcaact ttctcgtctg 540
acccttccgt tgccagatcc cggggggcca tggaaaagcc tgaactcacc gcgacgtctg 600
tcgagaagtt tctgatcgaa aagttcgaca gcgtctccga cctgatgcag ctctcggagg 660
gcgaagaatc tcgtgctttc agcttcgatg taggagggcg tggatatgtc ctgcgggtaa 720
atagctgcgc cgatggtttc tacaaagatc gttatgttta tcggcacttt gcatcggccg 780
cgctcccgat tccggaagtg cttgacattg gggaattcag cgagagcctg acctattgca 840
tctcccgccg tgcacagggt gtcacgttgc aagacctgcc tgaaaccgaa ctgcccgctg 900
ttctgcagcc ggtcgcggag gccatggatg cgatcgctgc ggccgatctt agccagacga 960
gcgggttcgg cccattcgga ccgcaaggaa tcggtcaata cactacatgg cgtgatttca 1020
tatgcgcgat tgctgatccc catgtgtatc actggcaaac tgtgatggac gacaccgtca 1080
gtgcgtccgt cgcgcaggct ctcgatgagc tgatgctttg ggccgaggac tgccccgaag 1140
tccggcacct cgtgcacgcg gatttcggct ccaacaatgt cctgacggac aatggccgca 1200
taacagcggt cattgactgg agcgaggcga tgttcgggga ttcccaatac gaggtcgcca 1260
acatcttctt ctggaggccg tggttggctt gtatggagca gcagacgcgc tacttcgagc 1320
ggaggcatcc ggagcttgca ggatcgccgc ggctccgggc gtatatgctc cgcattggtc 1380
ttgaccaact ctatcagagc ttggttgacg gcaatttcga tgatgcagct tgggcgcagg 1440
gtcgatgcga cgcaatcgtc cgatccggag ccgggactgt cgggcgtaca caaatcgccc 1500
gcagaagcgc ggccgtctgg accgatggct gtgtagaagt actcgccgat agtggaaacc 1560
gacgccccag cactcgtccg agggcaaagg aatagagcgg ccgcccggct gcagatcgtt 1620
caaacatttg gcaataaagt ttcttaagat tgaatcctgt tgccggtctt gcgatgatta 1680
tcatataatt tctgttgaat tacgttaagc atgtaataat taacatgtaa tgcatgacgt 1740
tatttatgag atgggttttt atgattagag tcccgcaatt atacatttaa tacgcgatag 1800
aaaacaaaat atagcgcgca aactaggata aattatcgcg cgcggtgtca tctatgttac 1860
tagatccgat gataagctgt caaacatgag gcctgagtgg ccgagctc 1908

## Beschreibung

Die vorliegende Erfindung betrifft ein neues Verfahren zum Herstellen von Deletionsmutanten, das auf einer PCR (Polymerase Chain Reaction)-Strategie basiert. Die Erfindung betrifft außerdem eine universell einsetzbare Knockout-Kassette, die ein selektierbares Marker-Gen enthält und durch das spezifische Design der Restriktionsschnittstellen charakterisiert ist, sowie ein Verfahren zum Herstellen dieser Knockout-Kassette.

Für die funktionelle Analyse von Genen ist es in vielen Fällen notwendig, die entsprechenden Gene zu deletieren bzw. gegen modifizierte Gene auszutauschen. Die klassische Methodik zur Herstellung von Deletionsmutanten zum Beispiel bei dem Basidiomyceten *Ustilago maydis* ist die Transformation mit linearen DNA-Fragmenten (DNA = deoxyribonucleic acid) (vgl. z.B. Bölker et al. 1995, Fotheringham et al. 1989, Kronstad et al. 1989, Tsukuda et al. 1988, Wang et al. 1988). Diese DNA-Fragmente enthalten ein selektierbares Marker-Gen (z.B. *hph, cbx, phleo*), das von Bereichen flankiert ist, die zum Integrationsort homolog sind. Über diese Flanken kommt es erfahrungsgemäß bei 10% bis 50% der Transformanden zur homologen Rekombination, was für den Austausch von Genen gezielt verwendet werden kann.

Die Herstellung von Knockout-Konstrukten für die Transformation erfolgt in der Regel über mehrere, zeitaufwendige Klonierungsschritte in Plasmidvektoren (vgl. z.B. Basse et al. 2000, Lehmler et al. 1997, Quadbeck-Seeger et al. 2000, Regenfelder et al. 1997). Da sichergestellt werden muss, dass die für die Rekombination notwendigen Bereiche das betreffende Gen flankieren, ohne mit dem offenen Leserahmen zu überlappen, sind oftmals mehrere PCR-Schritte für die Konstruktion der Knockout-Konstrukte notwendig. Vor der Transformation in das Genom des untersuchten Organismus werden diese Plasmidvektoren in der Regel mit einem Restriktionsenzym linearisiert, das im Plasmidrückgrat - also außerhalb des interessierenden Inserts - schneidet.

Die bisher bekannten Techniken zur Herstellung von Knockout-Mutanten besitzen also zum Einen den Nachteil, dass für jedes einzelne Gen, das deletiert werden soll, ein eigenes Knockout-Konstrukt in einem Plasmidvektor hergestellt werden muss. Zum Zweiten umfasst dieses Herstellungsverfahren eine Vielzahl von zeitaufwändigen Verfahrensschritten, die die Verknüpfung der flankierenden Bereiche des interessierenden Gens mit dem Marker-Gen umfasst, wobei sichergestellt sein muss, dass die flankierenden Bereiche nicht mit dem offenen Leseraster (ORF) des auszuschaltenden Gens überlappen und der ORF des Marker-Gens im Plasmidvektor intakt ist. Als Konsequenz der genannten Nachteile ist es nicht möglich, mit den bekannten Verfahren Deletionsmutanten im Hochdurchsatz herzustellen.

Eine *Deletionsmutante* im Sinne der vorliegenden Erfindung ist eine Mutante, bei der ein komplettes Gen deletiert und/oder gegen ein modifiziertes Gen ausgetauscht wurde. Eine *Knockout-Mutante* ist demnach eine Deletionsmutante, bei der ein zu untersuchendes Gen durch Deletion und/oder Genaustausch ausgeschaltet wurde. Die Begriffe *Deletionsmutante* und *Knockout-Mutante* werden in der Beschreibung synonym verwendet.

DNA-Konstrukte, die für die Transformation und zum Herstellen der Deletionsmutanten eingesetzt werden, werden im Folgenden als *Knockout-Konstrukte* bezeichnet.

Unter einer *Knockout-Kassette* wird in der vorliegenden Beschreibung ein Marker-Gen einschließlich Promotor und Terminator verstanden, das an beiden Flanken speziell konstruierte Schnittstellen für ein Restriktionsenzym besitzt.

Ein *Marker-Gen* im Sinne der Erfindung ist ein Gen, das den transformierten Zellen durch Expression des entsprechenden Genproduktes einen Selektionsvorteil verschafft (z.B. einen Wachstumsvorteil). Marker-Gene codieren beispielsweise für Enzyme, die eine Resistenz gegen bestimmte Antibiotika bedingen.

Unter *Transformation* soll im Rahmen der Erfindung ein einfacher genetischer Transfer verstanden werden. Das "nackte", lineare Knockout-Konstrukt penetriert ohne Beteiligung einer weiteren Trägersubstanz oder Trägerstruktur die Membran (und die eventuell vorhandene Zellwand) einer Rezipientenzelle und wird mit Hilfe der genetischen Rekombination in die Rezipienten-DNA eingebaut.

Als *Rezipientenzelle* werden im Rahmen der vorliegenden Erfindung alle Zellen verstanden, die durch Transformation in der Lage sind, (Fremd-)DNA aufzunehmen.

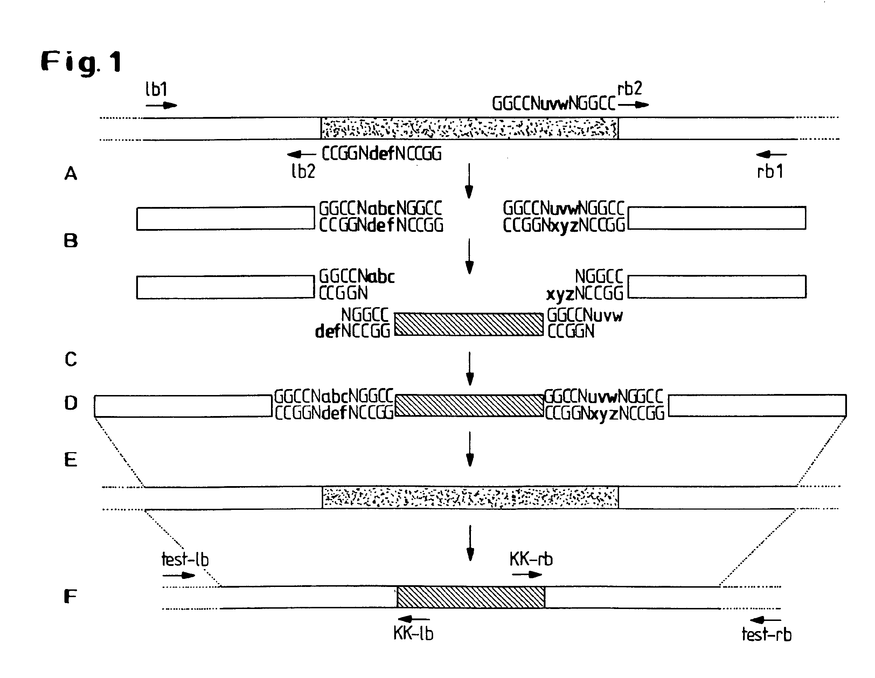

Unter einem *Primer* wird im Folgenden ein Starter-Oligonucleotid (DNA oder RNA, RNA = Ribonucleic acid) für die PCR verstanden. Als *Außenprimer* werden dabei die Primer verstanden, die als Starter-Oligonucleotid für die vom zu untersuchenden Gen weiter entfernt liegenden Ränder eingesetzt werden. Als *Innenprimer* werden dementsprechend diejenigen Primer verstanden, die als Starter-Oligonucleotid für die direkt an das zu untersuchende Gen angrenzenden Bereiche verwendet werden. In 5'-3'-Richtung geschrieben werden mit lb die Primer für die "linke Flanke" (lb = left border) des zu untersuchenden Gens bezeichnet, mit rb die entsprechenden Primer für die "rechte Flanke" (rb = right border). Die beiden Außenprimer sind nach dieser Definition lb1 und rb1, die beiden Innenprimer lb2 und rb2 (vgl. Abb. 1).

Die Aufgabe der vorliegenden Erfindung bestand in der Bereitstellung einer Methode, die die oben genannten Nachteile bekannter Verfahren überwindet, indem sie es ohne aufwändige Klonierungsschritte erlaubt, Konstrukte für den homologen Genaustausch direkt zu generieren.

Die Aufgabe wird durch die Bereitstellung eines PCR-basierten Verfahrens gelöst, das ein Knockout-Konstrukt liefert, welches für die homologe Rekombination zum Erzeugen von Deletionsmutanten direkt verwendet werden kann. Die Lösung der Aufgabe umfasst ebenfalls das Herstellen und Bereitstellen von Knockout-Kassetten, die im erfindungsgemäßen Verfahren universell für jedes interessierende Gen eingesetzt werden können.

Das erfindungsgemäße Verfahren bietet daher den Vorteil, Deletionsmutanten im Hochdurchsatz herzustellen. Dies ist insbesondere wichtig in Verfahren zum Auffinden von essentiellen Genen oder solchen Genen, die für die Pathogenese von Bedeutung sind, um neue Targets zur Wirkstoffsuche identifizieren zu können.

Gegenstand der vorliegenden Erfindung ist ein PCR-basiertes Verfahren zum Herstellen von Deletionsmutanten, das dadurch gekennzeichnet ist, dass man
A) in einem ersten Verfahrensschritt die ein Gen flankierenden Bereiche durch PCR mit für diese Bereiche spezifischen Primern generiert, wobei über die beiden Innenprimer an den das Gen flankierenden Enden zwei unterschiedliche Schnittstellen für dasselbe Restriktionsenzym erzeugt werden (vgl. Abb. 1A),
B) in einem zweiten Verfahrensschritt die Amplifikationsprodukte mit dem für die Schnittstellen spezifischen Restriktionsenzym schneidet, wodurch zwei nicht identische überhängende Enden entstehen (vgl. Abb. 1B),
C) in einem dritten Verfahrensschritt eine Knockout-Kassette, die die entsprechenden kompatiblen Schnittstellen besitzt, zwischen die beiden flankierenden Bereiche ligiert (vgl. Abb. 1C),
D) in einem vierten Verfahrensschritt das Ligationsprodukt mit PCR unter Einsatz der Außenprimer aus dem ersten Verfahrensschritt reamplifiziert (vgl. Abb. 1D) und
E) in einem fünften Verfahrensschritt das Amplifikationsprodukt aus dem vierten Schritt in eine Rezipientenzelle transformiert (vgl. Abb. 1E).

Die Kontrolle der Transformation und der erfolgreichen homologen Rekombination erfolgt anschließend durch Ganz-Zell-PCR mit Primern, die außerhalb der für die Rekombination verwendeten Flanken liegen. Verwendet man gleichzeitig Primer, die für die Randbereiche der Knockout-Kassette spezifisch sind, erhält man nur dann ein Amplifikat, wenn es zu einem homologen Genaustausch gekommen ist (vgl. Abb. 1F).

Ob es sich bei dem ausgetauschten Gen um ein essentielles Gen handelt, kann anschließend aus den Ergebnissen verschiedener Wachstumsexperimente geschlossen werden. Im Verfahren werden bevorzugt diploide Zellen eingesetzt, die also jeweils zwei Kopien von jedem Gen besitzen. Die homologe Rekombination erfolgt in der Regel bei nur einer Kopie.

Bei haploiden Organismen können nach der Meiose die Meioseprodukte direkt in Wachstumsexperimenten auf die Expression des Resistenzgens getestet werden. Zuerst werden verschiedene haploide Zellen (Meioseprodukte) auf ihre Fähigkeit getestet, auf einem Normalmedium zu wachsen (Test 1). Anschließend werden die Zellen auf ihre Fähigkeit getestet, auf einem Medium zu wachsen, auf dem für das Wachstum das Genprodukt des Marker-Gens notwendig ist (Test 2). Auf diesem Medium werden nur diejenigen Organismen überleben, bei denen ein homologer Genaustausch stattgefunden hat und das Marker-Gen vorliegt.

Unter *Normalmedium* wird hierbei ein üblicherweise bekanntes Medium zur Anzucht des untersuchten Organismus verstanden, z.B. YEPS-Medium (siehe Beispiele) für *Ustilago maydis*-Kulturen.

Unter einem *Selektionsmedium* wird ein Wachstumsmedium verstanden, auf dem ein untersuchter Organismus nur dann wachsen kann, wenn er das Genprodukt des Marker-Gens exprimieren kann. Als Beispiel für ein Selektionsmedium sei YEPS-Medium genannt, das zusätzlich zu den Standardkomponenten ein Antibiotikum (z.B. Hygromycin) enthält.

Zur Bestimmung des Wachstumsphänotypen lässt man den untersuchten Organismus oder Zellen von diesem einerseits auf Normalmedium (Test 1) und andererseits auf Selektionsmedium (Test 2) wachsen und vergleicht die Ergebnisse im Wachstumsverhalten.

Wurde durch das erfindungsgemäße Verfahren ein essentielles Gen ausgeschaltet, werden in Test 1 nur diejenigen Organismen wachsen können, bei denen das Gen noch vorhanden ist. Dagegen wird man bei Test 2 keine lebensfähigen Organismen erwarten.

Bei diploiden Organismen kann der Test nicht direkt mit den haploiden Meioseprodukten durchgeführt werden. 33% der aus den haploiden Meioseprodukten entstandenen diploide Folgegeneration muß, wenn es sich bei dem ausgetauschten Gen nicht um ein essentielles Gen handelt, zwei Kopien des Resistenzgens enthalten und entsprechend keine Kopie des ausgetauschten Gens. Die Präsenz des Resistenzgens kann entsprechend der Beschreibung für haploide Organismen nachgewiesen werden. Zusätzlich muß jedoch der Verlust des ausgetauschten Gens durch PCR-Techniken dokumentiert werden.

Werden keine Zellen bzw. Organismen gefunden, die zwei Kopien des Resistenzgens tragen, ist es wahrscheinlich, daß es sich bei dem ausgetauschten Gen um ein essentielles Gen handelt.

In gleicher Weise kann das Verfahren dazu genutzt werden, Gene zu identifizieren, die für bestimmte Eigenschaften (z.B. für die Pathogenität) des untersuchten Organismus wichtig sind.

Die vorliegende Erfindung betrifft demnach auch ein Verfahren zum Auffinden essentieller Gene und solcher Gene, die für bestimmte Eigenschaften des untersuchen Organismus wichtig sind, das dadurch gekennzeichnet ist, dass man die oben genannten Verfahrensschritte (A) bis (E) des erfindungsgemäßen Verfahrens ausführt und anschließend den Wachstumsphänotyp wie oben beschrieben bestimmt.

Essentielle Gene und beispielsweise Gene, die für die Pathogenität eines Organismus wichtig sind, können neue und interessante Targets bei der Suche nach neuen Wirkstoffen darstellen.

Die vorliegende Erfindung betrifft daher auch ein Verfahren zum Identifizieren von Targets für die Wirkstoffsuche, das dadurch gekennzeichnet ist, dass man zunächst ein essentielles Gen identifiziert und anschließend Ansätze entwickelt, die Expression des Gens zu verhindern oder die Funktion des Genprodukts zu inhibieren.

Aufgrund der vielseitig einsetzbaren Knockout-Kassette, können mit dem erfindungsgemäßen Verfahren Deletionsmutanten im Hochdurchsatz hergestellt werden.

Die vorliegende Erfindung betrifft daher auch ein Verfahren zum Herstellen von Deletionsmutanten im Hochdurchsatz.

Das beschriebene Verfahren zum Herstellen von Deletionsmutanten ist nicht auf einen bestimmten Zelltyp beschränkt. Es können sowohl prokaryotische wie eukaryotische Zellen verwendet werden. Bevorzugt wendet man das Verfahren bei eukaryotischen Zellen an, besonders bevorzugt bei Pilzen (Fungi), ganz besonders bevorzugt bei Basidiomycota und Ascomycota, insbesondere ganz besonders bevorzugt bei Ustilagomycetes, wie z.B. *Ustilago maydis*, oder Hemiascomycetes, wie z.B. Saccharomyces cerevisiae. Herausragende Bedeutung kommt dem erfindungsgemäßen Verfahren bei der Untersuchung von phytopathogenen Organismen wie z.B. dem Brandpilz *Ustilago maydis* zu.

Sofern die flankierenden Bereiche eines Gens bekannt sind, kommen alle Gene, für die diese Voraussetzung erfüllt ist, für das erfindungsgemäße Verfahren infrage. Die im ersten Verfahrensschritt durch PCR amplifizierten flankierenden Bereiche können in ihrer Größe variieren. Sie liegen im allgemeinen zwischen 60 bp und 2000 bp. Bevorzugt liegt die Größe der Flanken zwischen 600 bp und 1500 bp, ganz besonders bevorzugt zwischen 800 bp und 1200 bp.

Die im ersten Schritt des erfindungsgemäßen Verfahrens zur PCR benötigten spezifischen Außenprimer lb1 und rb1 werden bevorzugt so gewählt, dass jeweils vor dem 5'-Ende des Primers im genomischen Kontext ein Thymidin (T) liegt. Da durch die Taq-Polymerase (DNA-Polymerase aus dem thermophilen Bakterium **T**hermus **aq**uaticus) bei der Synthese des Gegenstrangs an dieser Position ein Adenosin (A) angehängt wird, kommt es zu keiner Veränderung der Sequenz der homologen Bereiche. Die Ligation der äußeren, durch die Primer lb1 und rb1 begrenzten, nicht mit einer Restriktionsendonuclease geschnittenen Enden der Moleküle wird darüber hinaus verhindert; denn erstens besitzen sie bedingt durch die Primermoleküle keine Phospatgruppe und zweitens liegen bedingt durch ein von der Taq-Polymerase bei der Synthese des Gegenstranges angehängtes Adenosin (A) keine kompatiblen Enden vor.

Die im ersten Schritt des erfindungsgemäßen Verfahrens zur PCR benötigten Innenprimer lb2 und rb2 werden bevorzugt so konstruiert, dass an den das Gen flankierenden Enden zwei unterschiedliche Schnittstellen für dasselbe Restriktionsenzym erzeugt werden. Bevorzugt setzt man Restriktionsenzyme ein, die eine definierte Erkennungssequenz von mindestens 6 bp (bp = Basenpaare), besonders bevorzugt von mindestens 8 bp, besitzen, die durch eine variable Sequenz von bevorzugt 3 bp bis 7 bp, besonders bevorzugt 3 bp bis 5 bp, ganz besonders bevorzugt 5 bp, unterbrochen ist und die die DNA innerhalb der variablen Sequenz so schneiden, dass überhängende Enden entstehen.

Im Folgenden werden einige Beispiele für geeignete Restriktionsenzyme genannt. In der Darstellung der Sequenz bedeuten A = Adenin, C = Cytosin, G = Guanin, T = Thymidin und N = eine beliebige Base. Pfeile geben die Restriktionsstelle an.

Beispielhaft genannt seien folgende Enzyme mit einer Erkennungssequenz von 6 bp und einer variablen Sequenz von 3 bp:

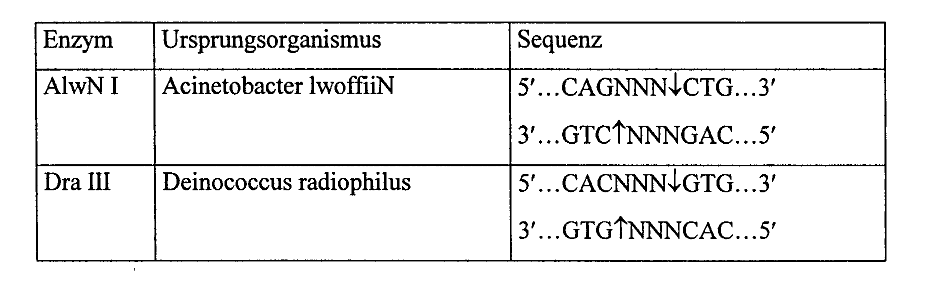

Beispielhaft genannt seien folgende Enzyme mit einer Erkennungssequenz von 6 bp und einer variablen Sequenz von 5 bp:

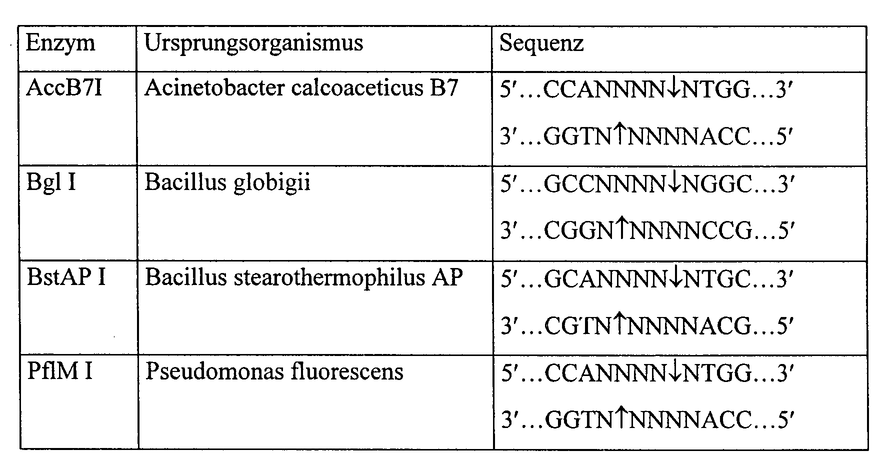

Beispielhaft genannt sei folgendes Enzym mit einer Erkennungssequenz von 6 bp und einer variablen Sequenz von 6 bp:

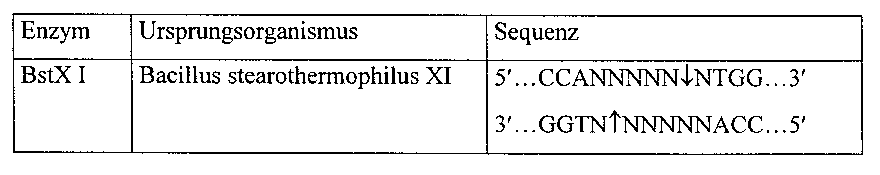

Beispielhaft genannt sei folgendes Enzym mit einer Erkennungssequenz von 8 bp und einer variablen Sequenz von 5 bp:

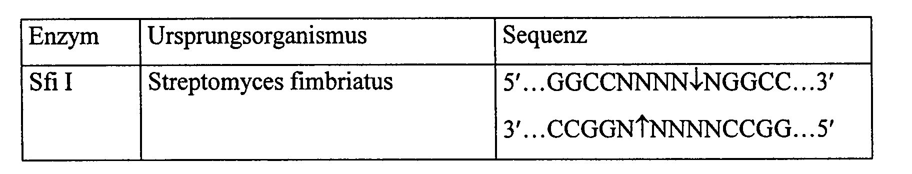

Durch die Wahl unterschiedlicher Sequenzen, die an die Primer lb2 und rb2 zur Erzeugung der Restriktionssequenz angehängt werden, kann erreicht werden, dass zwei unterschiedliche überhängende Enden entstehen, die nicht miteinander kompatibel sind.

Verwendet man beispielsweise die in der folgenden Tabelle gezeigten Sequenzen A und B als Schnittstellen für das Restriktionsenzym Sfi I erhält man die angegebenen ungleichen Enden nach Restriktion:

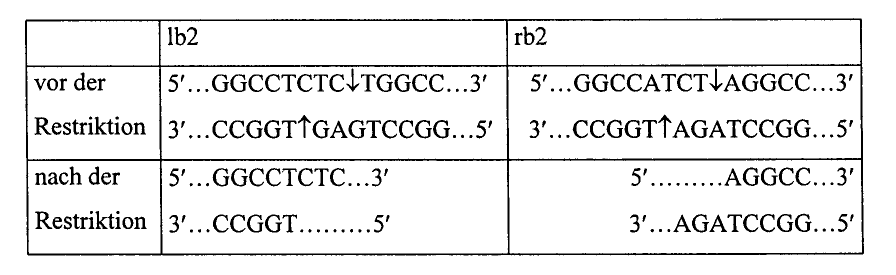

Die im zweiten Schritt des erfindungsgemäßen Verfahrens benötigten Restriktionsenzyme wurden bereits bei der Beschreibung der Sequenzauswahl der Primer im ersten Verfahrensschritt beschrieben.

Die im dritten Schritt des erfindungsgemäßen Verfahrens benötigte Knockout-Kassette enthält bevorzugt ein Marker-Gen für eine Resistenz gegen Antibiotika, Insektizide, Herbizide oder Fungizide, besonders bevorzugt ein Marker-Gen für eine Resistenz gegen Antibiotika, ganz besonders bevorzugt ein Marker-Gen für eine Resistenz gegen die Antibiotika Hygromycin (z.B. das Hygromycin-Phosphotransferase-Gen, *hph*), Carboxin (z.B. das Gen für die Eisen-Schwefel-Untereinheit der Succinat-Dehydrogenase, *cbx*) oder Phleomycin (Phleomycin-Resistenzgen, *phleo*), insbesondere ganz besonders bevorzugt ein Marker-Gen für eine Resistenz gegen das Antibiotikum Hygromycin.

Die im dritten Schritt des erfindungsgemäßen Verfahrens benötigte Knockout-Kassette wird bevorzugt so konstruiert, dass sie nach Linearisierung an beiden Enden kompatible Schnittstellen desselben Restriktionsenzyms besitzt, die durch die Primer lb2 und rb2 an den flankierenden Enden des auszutauschenden Gens erzeugt wurden.

Dadurch wird gewährleistet, dass nur drei Ligationsprodukte entstehen können:
- Knockout-Kassette mit der linken Flanke,
- Knockout-Kassette mit der rechten Flanke,
- Knockout-Kassette mit beiden Flanken.

Die Ligation erfolgt mit bekannten, kommerziell erhältlichen Ligasen und wird nach Angaben des Herstellers durchgeführt.

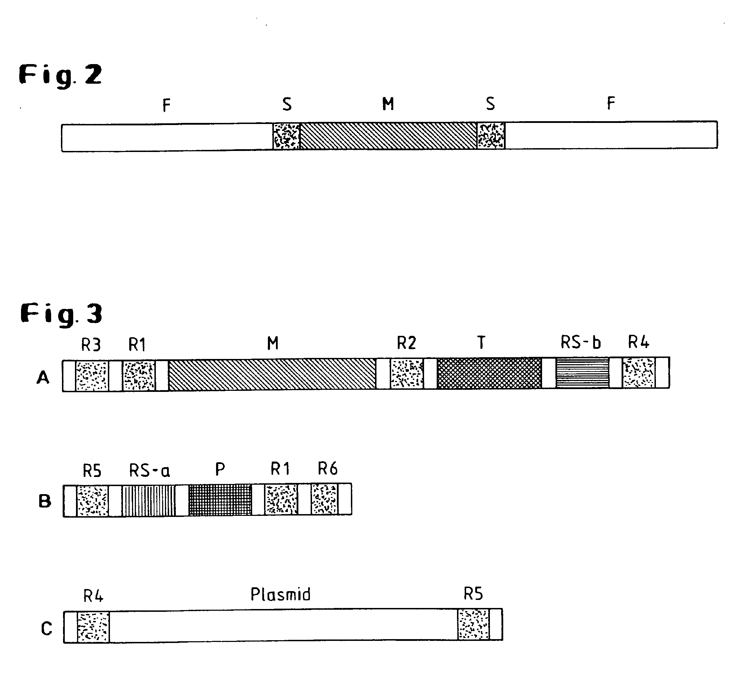

Die Reamplifizierung der Ligationsprodukte aus Schritt 3 des Verfahrens erfolgt im vierten Schritt des erfindungsgemäßen Verfahrens bevorzugt unter Einsatz derselben Primer lb1 und rb1, die bereits im ersten Verfahrensschritt verwendet wurden. Dabei können nur die Ligationsprodukte amplifiziert werden, die die Knockout-Kassette mit beiden Flanken besitzen. Man erhält also ein lineares DNA-Fragment, das an beiden Enden die beiden Bereiche enthält, die das gewünschte Gen flankieren. In der Mitte befindet sich das Marker-Gen, das an beiden Seiten jeweils über eine Restriktionsschnittstelle mit den flankierenden Bereichen verbunden ist (schematische Darstellung in Abb. 2).

Die Transformation in Schritt 5 des erfindungsgemäßen Verfahrens kann nach üblichen Standardtechniken zur DNA-Transformation erfolgen. Generell verwendbar sind beispielsweise Techniken zur Transformation von Protoplasten, Ultraschalltechniken, Makro- und Mikroinjektionen, Elektroporation, Dichtegradienten-Verfahren. Bevorzugt erfolgt die Transformation von Protoplasten des Schadpilzes *Ustilago maydis*.

Dabei geht man beispielsweise so vor, dass man zunächst Protoplasten von *U. maydis* herstellt. Dazu lässt man eine Zellkultur von *U. maydis* bis zu einer bestimmten Zelldichte (ca. 5 × 10⁷, entspricht einer optischen Dichte bei 600 nm von OD₆₀₀ = 0.6 bis 1.0) anwachsen. Anschließend zentrifugiert man die Zellen ab, resuspendiert das Pellet in einem Natriumcitrat/Sorbit-Puffer (SCS-Puffer). Nach erneutem Zentrifugieren nimmt man in SCS-Puffer mit Novozym auf. Die Protoplastierung erfolgt bei Raumtemperatur. Die Protoplasten werden anschließend mehrfach mit SCS-Puffer gewaschen und schließlich mit einem Tris/Sorbit/Calciumchlorid-Puffer (STC-Puffer) gewaschen und in diesem Puffer schließlich gelagert. Die Transformation erfolgt anschließend durch Mischen der hergestellten Protoplasten mit dem aus Schritt (D) des erfindungsgemäßen Verfahrens erhaltenen linearen DNA-Fragment mit der Knockout-Kassette. Zugabe von Polyethylenglykol stimuliert den Transfer der DNA in die Protoplasten durch Erhöhung der Permeabilität der Protoplastenmembran (vgl. Tsukuda et al. 1988).

Die Kontrolle der Transformation und der erfolgreichen homologen Rekombination erfolgt bevorzugt mit Ganz-Zell-PCR. Dabei werden vorzugsweise Primer eingesetzt, die zu Bereichen außerhalb der verwendeten flankierenden Genbereiche komplementär sind (z.B. test-lb und test-rb, siehe Abb. 1F). Kombiniert man diese Testprimer mit Primern für Bereiche innerhalb der Knockout-Kassette (KK-lb und KK-rb) erhält man nur bei erfolgreichem Genaustausch die entsprechenden Amplifikate.

### Konstruktion einer Knockout-Kassette

Im erfindungsgemäßen Verfahren wird im dritten Verfahrensschritt (C) eine Knockout-Kassette benötigt. Diese Knockout-Kassette zeichnet sich dadurch aus, dass sie zum Knockout beliebiger Gene verwendet werden kann. Denn sie wird so konstruiert, dass sie von zwei verschiedenen Restriktionsschnittstellen flankiert wird, wodurch die Orientierung der Nucleotidsequenz festgelegt ist und die Kombination mit entsprechend vorbereiteten Flanken eines beliebigen Gens möglich wird.

Die vorliegende Erfindung betrifft daher auch eine Knockout-Kassette, die eine erste nicht-palindromische Restriktionsschnittstelle, ein Marker-Gen mit Promotor und Terminator, sowie eine zweiten nicht-palindromische Restriktionsschnittstelle und gegebenenfalls weitere palindromische Restriktionsschnittstellen enthält.

Außerdem wird erfindungsgemäß die Verwendung dieser Knockout-Kassette im Verfahren zum Herstellen von Deletionsmutanten beansprucht.

Weiterhin betrifft die vorliegende Erfindung also ein Verfahren zum Herstellen einer Knockout-Kassette, das dadurch gekennzeichnet ist, dass man
a) in einem ersten Schritt aus einem Plasmid I mit den Primern 1 und 2 ein Marker-Gen amplifiziert, wodurch an beiden Enden des Marker-Gens unterschiedliche Restriktionsschnittstellen R1 und R2 eingeführt werden, und das PCR-Produkt anschließend mit den Enzymen für diese Schnittstellen R1 und R2 restringiert, und
b) in einem folgenden Schritt das so erhaltene PCR-Produkt mit einem Terminator verknüpft, indem man ein Plasmid II mit den Enzymen für die Schnittstellen R1 und R2 restringiert und das durch die Restriktion freigesetzte Fragment gegen das PCR-Fragment mit dem Marker-Gen aus Schritt (a) austauscht, wodurch man ein Plasmid III erhält, und
c) ausgehend von diesem Plasmid III in einer zweiten PCR mit den Primern 1 und 4 das Marker-Gen zusammen mit dem Terminator amplifiziert, wodurch am 5'-Ende wenigstens die Schnittstelle R1 und am 3'-Ende die Schnittstelle RS-b gefolgt von einer Schnittstelle R4 generiert werden, und das so erhaltene Fragment A anschließend in ein Plasmid IV kloniert, wodurch man Plasmid V erhält, und
d) ausgehend von Plasmid I in einer dritten PCR durch die Primer 5 und 6 eine funktionsfähige Promotorsequenz für das Marker-Gen amplifiziert, wodurch am 5'-Ende der Promotor-Sequenz die Schnittstellen R5 und RS-a und am 3'-Ende wenigstens die Schnittstelle R1 generiert werden, und das so erhaltene Fragment B anschließend in ein Plasmid IV kloniert, wodurch man das Plasmid VI erhält, und
e) in einem letzten Schritt i) das Fragment A durch Restriktion des Plasmids V mit den Enzymen R1 und R4 freisetzt, ii) das Fragment B durch Restriktion des Plasmids VI mit den Enzymen R5 und R1 freisetzt, iii) das Fragment C durch Restriktion eines Plasmids VII mit den Enzymen R5 und R4 freisetzt und iv) die drei erhaltenen Fragmente zusammen ligiert, wodurch man ein Plasmid VIII erhält, und
f) das Plasmid VIII aus Schritt (e) mit dem Restriktionsenzym für die Schnittstellen RS-a und RS-b schneidet.

Das im Verfahrensschritt (a) verwendete Plasmid I ist dadurch charakterisiert, dass es zum einen ein Marker-Gen und zum anderen eine Sequenz für einen entsprechenden funktionsfähigen Promotor enthält.

Das im Verfahrensschritt (b) verwendete Plasmid II ist dadurch charakterisiert, dass es zum einen eine Terminator-Sequenz und zum anderen eine austauschbare Gensequenz enthält.

In den Verfahrensschritten (c) und (d) wird jeweils ein Plasmid IV verwendet, in das durch ein Standardverfahren bestimmte Fragmente kloniert werden. Als Standardverfahren kommt z.B. die TOPO-Klonierung mit einem Kit der Fa. Invitrogen infrage, der als Plasmid IV beispielsweise das Plasmid pCR2.1 enthält (vgl. Tsukuda et al. 1988 und Informationen des Herstellers).

Das in Verfahrensschritt (e) verwendete Plasmid VII ist dadurch charakterisiert, dass es mit denselben Restriktionsenzymen für die Schnittstellen R4 und R5 geschnitten werden kann, um anschließend mit den entsprechenden Enden der Fragmente A und B, die in Verfahrensschritt (e) freigesetzt werden, ligiert zu werden. Beispielhaft sei hier das Plasmid pBSKSII (Fa. Stratagene) als geeigneter Vektor genannt.

Die Restriktionsschnittstellen R1, R2, R4 und R5 sind unterschiedliche, palindromische Nucleotidsequenzen, die von unterschiedlichen Restriktionsenzymen erkannt und geschnitten werden.

Durch die Wahl der Primer 1 und 6 können gegebenenfalls mehrere hintereinander liegende Restriktionsschnittstellen gleichzeitig eingeführt werden.

Damit das erfindungsgemäße Verfahren zum Herstellen der Knockout-Kassette funktioniert, muss gewährleistet sein, dass die in Schritt (a) eingeführten Restriktionsschnittstellen R1 und R2 identisch sind mit den Schnittstellen des Plasmids II, mit denen die austauschbare Gensequenz entfernt werden kann.

Bei der Konstruktion einer Knockout-Kassette, die in Schritt (C) des erfindungsgemäßen Verfahrens benötigt wird, geht man in einer bevorzugten Ausführungsform beispielsweise wie folgt vor.
a) In einem ersten Schritt wird ein Plasmid I verwendet, das zum einen ein Marker-Gen (z.B. das bakterielle Hygromycin-Resistenzgen, *hph*) und zum anderen eine Sequenz für einen entsprechenden Promotor (z.B. *hsp70*-Promotor aus *U. maydis*) enthält (z.B. das Plasmid pCM54, vgl. Tsukuda et al. 1988). Aus diesem Plasmid I wird durch PCR mit geeigneten Primern 1 und 2 (z.B. den Primern hph-Nco/Bam und hph-Stop) zunächst das Marker-Gen amplifiziert. Dadurch werden an beiden Enden des Marker-Gens unterschiedliche Restriktionsschnittstellen R1 (z.B. Schnittstelle des Restriktionsenzym Nco I mit der Sequenz 5'-C↓CATGG-3' am ATG-Startcodon des *hph*-Gens) und R2 (z.B. Schnittstelle des Restriktionsenzym Not I mit der Sequenz 5'-GC↓GGCCGC-3' ein Nucleotid hinter dem STOP-Kodon des *hph*-Gens) eingeführt (Abb. 3A). Das PCR-Produkt wird anschließend mit den Enzymen für die Schnittstellen R1 (z.B. das Enzym Nco I) und R2 (z.B. das Enzym Not I) restringiert.
b) In einem folgenden Schritt wird dieses PCR-Produkt, das das Marker-Gen enthält, mit einem Terminator verknüpft. Dazu wird ein zweites geeignetes Plasmid II (z.B. das Plasmid potefSG, vgl. Spellig et al. 1996) verwendet. Dieses Plasmid II enthält zum einen die Terminator-Sequenz und zum zweiten eine austauschbare Gensequenz, die mit Enzymen für dieselben Schnittstellen R1 und R2 restringiert werden kann. Plasmid II wird also mit den Enzymen für die Schnittstellen R1 (z.B. das Enzym Nco I) und R2 (z.B. das Enzym Not I) restringiert und das durch die Restriktion freigesetzte Fragment (z.B. mit dem sGFP-Gen) gegen das PCR-Fragment mit dem Marker-Gen (im Beispiel dem *hph*-Gen) ausgetauscht. Durch diese Klonierung wird das Marker-Gen am 3'-Ende durch den Terminator (z.B. dem Agrobakterium NOS-Terminator des Plasmids potefSG, NOS = Nopalin Synthetase) flankiert, um eine effektive Termination der Marker-Gen-Transkription zu gewährleisten. Dadurch erhält man ein Plasmid III.
c) In einer zweiten PCR wird, ausgehend von diesem Plasmid III, das Marker-Gen zusammen mit dem Terminator amplifiziert. Man verwendet dazu geeignete Primer 1 (z.B. hph-Nco/Bam) und Primer 4 (z.B. 3-hph). Durch diese PCR werden am 5'-Ende die Schnittstellen R3 (z.B. für das Restriktionsenzym BamHI mit der Sequenz 5'-G↓GATCC-3') und R1 (z.B. für das Restriktionsenzym Nco I) und am 3'-Ende die Schnittstelle RS-b (z.B. für das Enzym Sfi I) gefolgt von einer Schnittstelle R4 (z.B. für das Enzym Sac I mit der Sequenz 5'-GAGCT↓C-3') generiert. Man erhält so das in Abb. 3A schematisch dargestellte Fragment A. Das PCR-Produkt (Fragment A, Abb. 3A) wird anschließend direkt über ein Standardverfahren (z.B. TOPO-Klonierung mit einem Kit der Fa. Invitrogen) in ein Plasmid IV (z.B. das bekannte Plasmid pCR2.1) kloniert. Dadurch entsteht ein Plasmid V (z.B. das Plasmid pBS-hph-Nos).
d) In einer dritten PCR wird, ausgehend von dem Plasmid I (z.B. dem Plasmid pCM54, siehe oben), durch die Primer 5 (z.B. Primer 5-hsp) und 6 (z.B. Primer hsp-Nco) ein Fragment eines Promotors für das Marker-Gen (z.B. ein 550 bp langes Fragment des *U. maydis hsp70*-Promotors) amplifiziert. Durch diese PCR wird am 5'-Ende der Promotor-Sequenz eine Schnittstelle R5 (z.B. für das Restriktionsenzym XhoI mit der Sequenz 5'-C↓TCGAG-3') und direkt darauf folgend, die Schnittstelle RS-a (z.B. für das Enzym Sfi I) eingeführt. Am 3'-Ende wird eine Schnittstelle R1 (z.B. für das Enzym Nco I) gefolgt von einer Schnittstelle R6 (z.B. für das Enzym HindIII mit der Sequenz 5'-A↓AGCTT-3') generiert. Man erhält so das in Abb. 3B schematisch dargestellte Fragment B. Das PCR-Produkt (Fragment B, Abb. 3B) wird wiederum direkt über ein Standardverfahren (z.B. TOPO-Klonierung mit einem Kit der Fa. Invitrogen) in ein Plasmid IV (z.B. das bekannte Plasmid pCR2.1) kloniert. Dadurch entsteht ein Plasmid VI (z.B. das Plasmid pBS-hsp). Die korrekten Sequenzen der über PCR generierten Fragmente werden nach der Klonierung durch Sequenzierung verifiziert.
e) In einem letzten Schritt wird i) das Plasmid V mit den Enzymen R1 und R4 geschnitten, wodurch Fragment A (Abb. 3A) mit dem Marker-Gen, der Terminator-Sequenz und der Schnittstelle RS-b ausfällt, ii) das Plasmid VI mit den Enzymen R5 und R1 geschnitten, wodurch Fragment B (Abb. 3B) mit der Promotor-Sequenz und der Schnittstelle RS-a ausfällt, iii) ein geeignetes Plasmid VII (z.B. das käufliche Plasmid pBSKSII, Fa. Stratagene) mit R5 und R4 geschnitten, wodurch man ein Fragment C (Abb. 3C) erhält, und iv) die drei erhaltenen Fragmente gemeinsam ligiert, wodurch man ein Plasmid VIII mit der Knockout-Kassette erhält (schematische Darstellung siehe Abb. 4, z.B. das Plasmid pBS-hhn siehe Abb. 5).
f) Aus diesem Plasmid VIII kann die Knockout-Kassette durch Restriktion mit dem gewählten Restriktionsenzym für die Schnittstellen RS-a und RS-b gewonnen und im erfindungsgemäßen Verfahren zum Herstellen von Deletionsmutanten eingesetzt werden.

Entsprechend dieses beschriebenen Verfahrens wurde eine Knockout-Kassette hergestellt, die das Hygromycin-Phosphotransferase-Gen (*hph*) enthält (siehe Beispiele).

### Ustilago maydis

*Ustilago maydis* ist ein phytopathogener Basidiomycet, der *Zea mays* (Mais) und die verwandte Art *Euchlena mexicana* (Teosinte) befällt. Dieser Organismus stellt ein Modellsystem zur Untersuchung der phytopathogenen Entwicklung dar. Das erfindungsgemäße Verfahren wird deshalb ganz besonders bevorzugt zur Untersuchung der Genfunktionen bei diesem Organismus eingesetzt.

Bei infizierten Pflanzen kann man als erste Symptome Chlorosen und einen durch Anthocyane verursachte Verfärbung beobachten; später erfolgt die für die Krankheit charakteristische Bildung von Tumoren, die aus hypertrophiertem Pflanzengewebe und großen Mengen an pilzlichen Teliosporen bestehen. Diese Teliosporen werden durch Wind verbreitet und können so leicht zu Neuinfektionen führen.

Während des Lebenszyklus von *Ustilago maydis* können zwei morphologisch distinkte Phasen unterschieden werden. Die Keimung der diploiden Teliosporen führt zur Bildung der septierten Probasidie, von deren einzelnen Kompartimenten sukzessiv haploide Zellen, sogenannte Sporidien, abgeschnürt werden. In dieser haploiden Form vermehrt sich *U. maydis* durch hefeartige Knospung als Saprophyt. Die Fusion zweier Sporidien führt zu dramatischen morphologischen und physiologischen Veränderungen: das resultierende Dikaryon wächst nun filamentös und ist in der Lage, die Wirtspflanze zu infizieren. In der Pflanze wächst der Pilz nach der Penetration zunächst inter- als auch intrazellulär mit verzweigten, septierten Hyphen. Die Pflanze zeigt dabei zunächst keine sichtbaren Abwehrreaktionen. Zu einem späteren Zeitpunkt kommt es zu einer lokalen, massiven Proliferation des Pilzes, die mit der Bildung der Pflanzentumore einhergeht. Die Hyphen beginnen zu schwellen und winden sich zunehmend umeinander; zur etwa gleichen Zeit findet auch die Karyogamie statt. Aus diesen sporogenen Hyphen bilden sich letztlich die Teliosporen.

Die Fusion der Sporidien, die zur Bildung des pathogenen Dikaryons führt, wird genetisch durch die zwei Kreuzungstyp-Loci von *Ustilago maydis* kontrolliert. Zur Etablierung eines stabilen Dikaryons müssen die Sporidien sowohl verschiedene Allele des biallelischen *a*-Locus als auch verschiedene Allele des multiallelischen *b*-Locus tragen. Beiden Loci können während der Entwicklung unterschiedliche Funktionen zugeordnet werden. Der *a*-Locus kontrolliert die Zellerkennung und Zellfusion über ein Pheromon-Rezeptor System. Jedes der beiden *a*-Allele kodiert ein prenyliertes Peptid-Pheromon (Mfa1 in *a1* und Mfa2 in *a2*) sowie einen Rezeptor für das Pheromon des jeweils anderen Kreuzungstyps (Pra1 in *a1* und Pra2 in *a2*); zusätzlich enthält der *a2* Locus zwei Gene mit unbekannter Funktion, *lga2* und *rga2*.

Nach der Zellfusion wird über ein vom *b*-Locus kodiertes intrazellulären Selbst-Nichtselbst Erkennungssystem entschieden, ob die weiteren Schritte bei der sexuellen und pathogenen Entwicklung initiiert werden. Die Fähigkeit, die Wirtspflanze zu infizieren, ist unabhängig vom *a*-Locus; es ist hinreichend, wenn verschiedene Allele des *b*-Locus in einer Zelle gemeinsam vorliegen. Dem *b*-Locus kommt damit die Rolle des zentralen molekularen Schalters für die Pathogenese zu.

Der b-Locus kodiert für zwei unterschiedliche, divergent transkribierte Gene, bE und bW. Die von den Genen kodierten Proteine haben eine abgeleitete Größe von 473 Aminosäuren (bE) und 645 Aminosäuren (bW); die beiden Proteine zeigen keine Homologien zueinander, mit Ausnahme einer Homeodomäne, einer bei Eukaryonten hochkonservierte Domäne die sequenzspezifische Bindung an DNA vermittelt. Trotz der fehlenden Homologie zeigen die Proteine jedoch einen ähnlichen Gesamtaufbau. Sowohl bei bE als auch bei bW kann eine aminoterminale, variable Domäne, in der sich die allelischen Unterschiede der jeweiligen Proteine häufen, von einer konstanten, hochkonservierten carboxyterminalen Domäne unterschieden werden. Die Kreuzung von Mutanten, die Deletionen entweder in bE oder in bW Genen tragen, zeigte, daß es für die Initiation des pathogenen Programms ausreichend ist, wenn Stämme mit einem funktionellen bE und einem bW Gen kombiniert werden, solange die Gene von verschiedenen Allelen stammen.

Die Anwendbarkeit des erfindungsgemäßen Verfahrens wurde durch die Deletion des *b*-Kreuzungstyp-Locus gezeigt (siehe Beispiele).

### Beispiele

### Konstruktion der Hygromycin-Knockout-Kassette (hph-Kassette)

### Schritt 1

Das bakterielle Hygromycin-Resistenzgen (*hph*) wird aus dem Plasmid pCM54 (vgl. Tsukuda et al. 1988) durch PCR mit den Primern hph-Nco/Bam (SEQ ID NO. 11) und hph-Stop (SEQ ID NO. 12) amplifiziert (PCR-Protokoll nach Innis et al. 1990; Zyklen: a) 1 Zyklus von 10 min bei 94°C, b) 30 Zyklen von jeweils 1 min bei 94°C, 1 min bei 60°C, 3 min bei 72°C, c) 1 Zyklus von 10 min bei 72°C). Dadurch werden an beiden Flanken des *hph*-Gens unterschiedliche Restriktionsschnittstellen eingeführt: Nco I-Schnittstelle mit der Sequenz 5'-C↓CATGG-3' am ATG-Startcodon des *hph*-Gens und Not I-Schnittstelle mit der Sequenz 5'-GC↓GGCCGC-3' ein Nucleotid hinter dem STOP-Kodon des *hph*-Gens. Das PCR-Produkt wird anschließend mit den Enzymen Nco I und Not I (New England Biolabs, Bedingungen nach Angaben des Herstellers) restringiert.

### Schritt 2

In einem folgenden Schritt wird dieses PCR-Produkt, das das *hph*-Gen enthält, mit dem Agrobakterium NOS-Terminator verknüpft. Dazu wird das Plasmid potefSG (vgl. Spellig et al. 1996), welches zum Einen die Sequenz des Agrobakterium NOS-Terminators und zum Zweiten eine austauschbare Sequenz für das sGFP-Gen enthält, mit den Enzymen Nco I und Not I (New England Biolabs, Bedingungen nach Angaben des Herstellers) restringiert. Durch die Restriktion wird das Fragment mit dem sGFP-Gen freigesetzt und gegen das PCR-Fragment mit dem *hph-*Gen ausgetauscht. Durch diese Klonierung wird das *hph*-Gen am 3'-Ende durch den Agrobakterium NOS-Terminator flankiert, um eine effektive Termination der *hph*-Transkription zu gewährleisten.

### Schritt 3

In einer zweiten PCR wird, ausgehend von diesem Plasmid, das *hph*-Gen zusammen mit dem NOS-Terminator amplifiziert. Man verwendet dazu die Primer hph-Nco/Bam (SEQ ID NO. 11) und 3-hph (SEQ ID NO. 13) (PCR-Protokoll nach Innis et al. 1990; Zyklen: a) 1 Zyklus von 10 min bei 94°C, b) 30 Zyklen von jeweils 1 min bei 94°C, 1 min bei 60°C, 3 min bei 72°C, c) 1 Zyklus von 10 min bei 72°C). Durch diese PCR werden am 5'-Ende die Schnittstellen für die Restriktionsenzyme BamH I (Sequenz 5'-G↓GATCC-3') und Nco I und am 3'-Ende die Schnittstelle Sfi I-b gefolgt von einer Schnittstelle für das Enzym Sac I (Sequenz 5'-GAGCT↓C-3') generiert. Das PCR-Produkt wird anschließend direkt über TOPO-Klonierung (mit einem Kit der Fa. Invitrogen, Bedingungen nach Angaben des Herstellers) in das Plasmid pCR2.1 kloniert. Dadurch entsteht das Plasmid pBS-hph-Nos.

### Schritt 4

In einer dritten PCR wird, ausgehend von dem Plasmid pCM54 (siehe oben), durch die Primer 5-hsp (SEQ ID NO. 14) und hsp-Nco (SEQ ID NO. 15) ein 550 bp langes Fragment des *U. maydis hsp70*-Promotors amplifiziert (PCR-Protokoll nach Innis et al. 1990; Zyklen: a) 1 Zyklus von 10 min bei 94°C, b) 30 Zyklen von jeweils 1 min bei 94°C, 1 min bei 60°C, 3 min bei 72°C, c) 1 Zyklus von 10 min bei 72°C). Durch diese PCR wird am 5'-Ende der *hsp70*-Promotor-Sequenz eine Schnittstelle für das Restriktionsenzym Xho I (Sequenz 5'-C↓TCGAG-3') und direkt darauf folgend die Schnittstelle Sfi I-a eingeführt. Am 3'-Ende wird eine Schnittstelle für das Enzym Nco I gefolgt von einer Schnittstelle für das Enzym HindIII (Sequenz 5'-A↓AGCTT-3') generiert. Das PCR-Produkt wird wiederum direkt über ein TOPO-Klonierung (mit einem Kit der Fa. Invitrogen, Bedingungen nach Angaben des Herstellers) in das Plasmid pCR2.1 kloniert. Dadurch entsteht das Plasmid pBS-hsp. Die korrekten Sequenzen der über PCR generierten Fragmente werden nach der Klonierung durch Sequenzierung verifiziert (ABI 377 Automated Sequencer, universal und reverse primer, Bedingungen nach Herstellerangaben unter Verwendung der Standard Universal- und Reverse-Primer).

### Schritt 5

In einem letzten Schritt wird i) das Plasmid pBS-hph-Nos mit den Enzymen Nco I und Sac I geschnitten (New England Biolabs, Bedingungen nach Angaben des Herstellers), wodurch das Fragment mit dem *hph*-Gen, der NOS-Terminator-Sequenz und der Schnittstelle Sfi I-b ausfällt, ii) das Plasmid pBS-hsp mit den Enzymen Xho I und Nco I geschnitten (New England Biolabs, Bedingungen nach Angaben des Herstellers), wodurch das Fragment mit der *hsp70*-Promotor-Sequenz und der Schnittstelle Sfi I-a ausfällt, iii) das käufliche Plasmid pBSKSII (Fa. Stratagene) mit Xho I und Sac I geschnitten (New England Biolabs, Bedingungen nach Angaben des Herstellers), und iv) die drei erhaltenen Fragmente gemeinsam ligiert (Ligase der Fa. Roche, Bedingungen nach Angaben des Herstellers), wodurch man das Plasmid pBS-hhn erhält. Aus diesem Plasmid kann die Hygromycin-Knockout-Kassette durch Restriktion mit Sfi I gewonnen und im erfindungsgemäßen Verfahren eingesetzt werden.

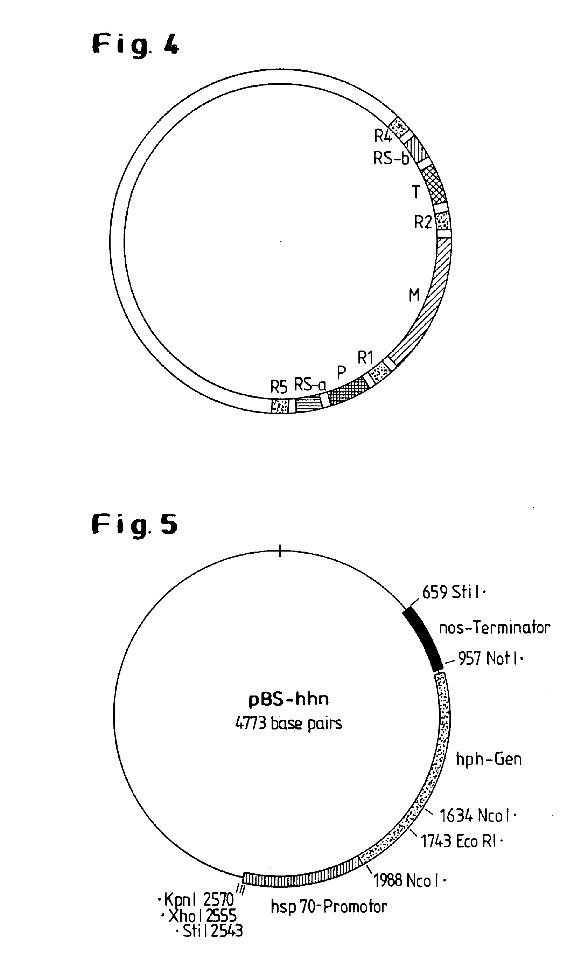

Die physikalische Karte des Plasmids pBS-hhn ist in Abb. 5 dargestellt. Die Sequenz des Inserts von der Xho I-Schnittstelle über den *hsp70*-Promotor, das *hph*-Gen, den NOS-Terminator bis zur Sac I-Schnittstelle besitzt die SEQ ID NO. 16.

### Deletion des b-Kreuzungstyp-Locus

Durch PCR werden je 1 kb lange Bereiche, die den *b*-Locus flankieren, unter Verwendung der Primer mit den Sequenzen lb1 (SEQ ID NO. 1), lb2 (SEQ ID NO. 2), rb1 (SEQ ID NO. 3)und rb2 (SEQ ID NO. 4) generiert.

Die PCR wird unter folgenden Bedingungen durchgeführt: Nach einem ersten Denaturierungsschritt von 10 min bei 94°C folgen 30 PCR-Zyklen von je 1 min bei 94°C (Denaturierung), 1 min bei 60°C (Hybridisierung) und 1 min bei 72°C (Polymerase-Reaktion), worauf abschließend 10 min bei 72°C inkubiert wird (PCR-Protokoll nach Innis et al. 1990).

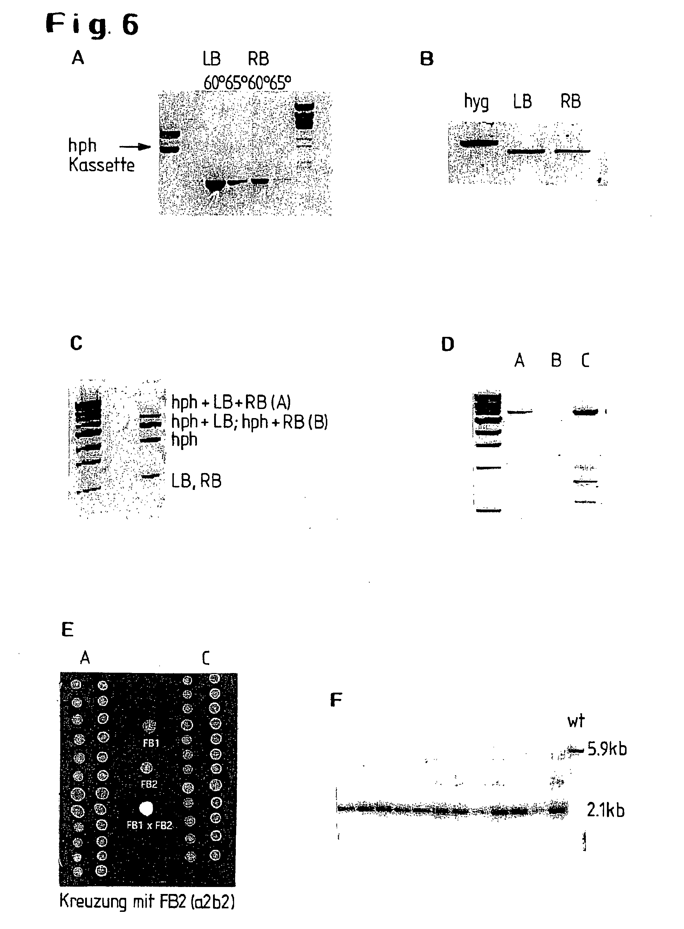

Durch die PCR Reaktion werden an den Enden der Fragmente, die an den *b*-Locus grenzen, zwei verschiedene zu den Sfi I Schnittstellen der *hph*-Kassette kompatible Sfi I Schnittstellen eingeführt (Abb. 6A). Der Primer lb2 enthält dafür neben der Erkennungssequenz für den inneren linken flankierenden Bereich die Sequenz für die Sfi I-b-Schnittstelle sowie 3 weitere Nucleotide. Der Primer rb2 enthält dafür neben der Erkennungssequenz für den inneren rechten flankierenden Bereich die Sequenz für die Sfi I-a-Schnittstelle sowie 3 weitere Nucleotide.

Der Vektor pBS-hhn und die PCR generierten Fragmente werden entsprechend mit Sfi I restringiert, gelelektrophoretisch aufgereinigt (Abb. 6B) und mit der *hph*-Kassette ligiert.

Die Restrikion der PCR-Fragmente erfolgt nach Ethanolfällung mit Sfi I (Fa. New England Biolabs, 20 Units, 2 h, Bedingungen der Enzymreaktion nach Herstellerangaben).

Die Reinigung der Fragmente erfolgt nach elektrophoretischer Auftrennung in Agarosegelen mit dem Gelelutions-Kit Jetsorb der Fa. Genomed nach Angaben des Herstellers.
Die Ligation der Flanken mit der *hph-*Kassette erfolgt in der Weise, dass man 0,2 µg jeder Flanke zusammen mit 0,2 µg der 2 kb langen *hph*-Kassette mit 2,5 Units Ligase (Fa. Roche, Bedingungen der Enzymreaktion nach Herstellerangaben) inkubiert.

Bedingt durch die nicht palindromischen, unterschiedlichen Überhänge der Sfi I Schnittstellen wird sowohl die Ligation von gleichen, als auch von verschiedenen Flanken verhindert. Eine Ligation über die stumpfen Enden der Fragmente wird ausgeschlossen, da die bei der PCR Reaktion eingestzten Primer nicht phosphoryliert sind. Abb. 6C zeigt, dass die Ligationsprodukte den Erwartungen entsprechen. Zusätzlich zu den Ausgangsprodukten entstehen zwei weitere Banden, die in ihrer Größe der *hph*-Kassette mit der linken Flanke oder rechten Flanke (ca. 3 kb, Bande B) bzw. der *hph*-Kassette mit beiden Flanken (ca. 4 kb, Bande A) entsprechen.

Wie in Abb. 6D dargestellt, wird in einer anschließenden PCR mit den entsprechenden Außenprimern nur das Produkt A amplifiziert. Dabei kann für diese Reaktion entweder das Ligationsprodukt (Spur C) direkt eingesetzt werden oder sicherheitshalber das gelelektrophoretisch aufgereinigte Produkt A (Spur A). Das Produkt B wird, wie erwartet, nicht amplifiziert (Spur B) (Abb. 6D).

Die PCR wird unter folgenden Bedingungen durchgeführt: Nach einem ersten Denaturierungsschritt von 10 min bei 94°C folgen 30 PCR-Zyklen von je 1 min bei 94°C (Denaturierung), 1 min bei 60°C (Hybridisierung) und 5 min bei 72°C (Polymerase-Reaktion), worauf abschließend 10 min bei 72°C inkubiert wird.

PCR Produkte aus den Ansätzen A und C werden gefällt, in Wasser aufgenommen und direkt zur Transformation des *Ustilago maydis* Stammes FB1 (*a1b1*) eingesetzt (Beschreibung der Transformation siehe unten). Von jedem Transformationsansatz werden 20 Kolonien gegen den kompatiblen Stamm FB2 (*a2b2*) gekreuzt; in keinem Fall kam es zu einer Ausbildung dikaryotischer Filamente, die die Kolonien weiß erscheinen lassen (vergleichbar mit der Kontrolle FB1 x FB2), was auf einen Verlust der *b*-Funktion bei allen getesteten Transformanden hinweist (Abb. 6E). Von je 6 Transformanden wurde eine Southern-Blot-Analyse durchgeführt; in allen Fällen war der *b*-Locus gegen die *hph*-Kassette ausgetauscht worden (Abb. 6F).

Zur schnelleren Überprüfung kann statt der Southern-Analyse eine PCR-Analyse durchgeführt werden. Dazu werden in einem Standard-PCR-Ansatz (PCR-Protokoll nach Innis et al. 1990) die DNA der Transformanden verwendet. Statt der DNA können alternativ zwischen 10⁴ und 10⁵ Zellen von *Ustilago maydis* (aus einer frisch gewachsenen Kultur, in sterilem Wasser gewaschen) zugesetzt werden. (Zyklen: a) 1 Zyklus von 10 min bei 94°C, b) 30 Zyklen von jeweils 1 min bei 94°C, 1 min bei 60°C, 2 min bei 72°C, c) 1 Zyklus von 10 min bei 72°C).

Die Primer-Kombinationen "KK-rb + rb1" sowie "KK-1b + 1b1" sollten auch bei ektopischen (nicht homologen) Integrationsereignissen PCR-Produkte liefern. Die Primer-Kombinationen "KK-1b + test-lb" sowie "KK-rb + test-rb" sollten nur bei homologer Rekombination PCR-Produkte ergeben. Die Primer test-lb und test-rb liegen 77 bp bzw. 70 bp außerhalb der durch die Primer lb1 und rb1 definierten Flanken der für die Rekombination verwendeten Knockout-Kassette.

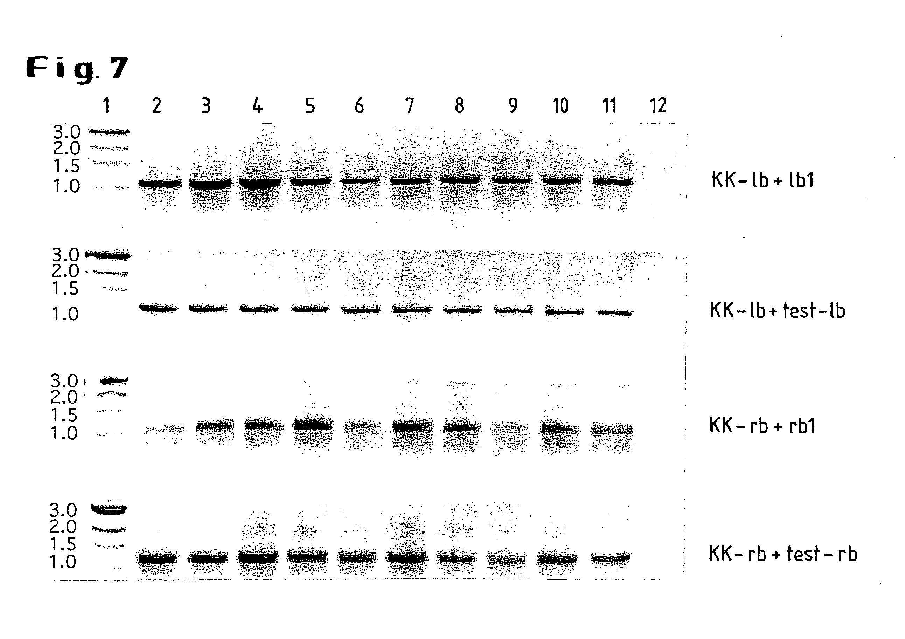

Die PCR-Reaktion mit den vier Primer-Kombinationen wurde für jeweils fünf der bereits durch Southern-Analyse charakterisierten Transformanden durchgeführt und die PCR-Produkte gelelektrophoretisch aufgetrennt. Wie erwartet traten in allen Fällen Banden der erwarteten Größe auf (KK-lb + lb1: 1044 bp; KK-1b + test-lb: 1121 bp; KK-rb + rb1: 1062 bp; KK-rb + test-rb: 1132 bp) (siehe Abb. 7).

### Herstellung von Protoplasten von U. maydis und Transformation

50 ml *U. maydis*-Kultur in YEPS Medium werden bei 28°C bis zu einer Zelldichte von ca. 5 × 10⁷/ml (OD₆₀₀ 0.6 bis 1.0) angezogen. Dazu wird eine stationäre Vorkultur in drei Schritten 1:100, 1:300, 1:1000 verdünnt und für ca. 16 Stunden bei 28°C und 200 rpm in einem Kulturkolben mit Schikanen inkubiert. Nach Erreichen der gewünschten Zelldichte wird die Kultur für 7 min bei 2500 g 2abzentrifugiert. Das Zellpellet wird in 25 ml SCS-Puffer resuspendiert und erneut für 7 min bei 2500 g zentrifugiert. Das Pellet wird in 2 ml SCS-Puffer mit 12,5 mg/ml Novozym 234 (z.B. Fa. NovoBiolabs) resuspendiert. Die Protoplastierung erfolgt bei Raumtemperatur und wird mikroskopisch alle 5 min verfolgt. Die Protoplasten werden anschließend mit 10 ml SCS-Puffer gemischt und bei 1100 g für 10 min zentrifugiert. Der Überstand wird verworfen. Dreimal wird das Pellet vorsichtig in jeweils 10 ml SCS-Puffer resuspendiert und zentrifugiert. Das Pellet wird anschließend mit 10 ml STC-Puffer gewaschen und daraufhin in 500 µl kaltem STC-Puffer resuspendiert und auf Eis gehalten.

Zu maximal 10 µl linearer DNA (zwischen 3 µg und 5 µg) werden nacheinander 15 µg Heparin und 50 µl Protoplasten (in STC-Puffer) zugegeben und 10 min auf Eis gekühlt. Anschließend werden 500 µl PEG3350 [40% (w/w) in STC-Puffer] zugesetzt, vorsichtig mit der Protoplastensuspension gemischt und für 15 min auf Eis inkubiert.

Zur Identifizierung von Transformanden wird der Transformationsansatz auf Agar-Platten ausplattiert (YEPS-Medium mit 1,5% Agar, 1 M Sorbitol und Antibiotikum; kurz vor dem Ausplattieren wird diese Agarschicht mit dem gleichen Volumen noch flüssigem Medium für Agar-Platten, das aber kein Antibiotium enthält überschichtet). Das Ergebnis wird nach 3 bis 4 Tagen Inkubation bei 28°C bestimmt.

### YEPS-Medium (vgl. Tsukuda et al. 1988)

1 % Hefeextrakt, 2 % Bactopepton (Difco), 2 % Saccharose in Wasser.

### SCS-Puffer

20 mM Natriumcitrat, 1,0 M Sorbit in Wasser, pH 5,8.

### STC-Puffer

10 mM Tris/HCl (pH 7,5), 1,0 M Sorbit, 100 mM CaCl₂ in Wasser.
Medium für Agar-Platten zur Transformationskontrolle
YEPS-Medium mit 1,5 % Agar, 1,0 M Sorbitol und Antibiotikum.

Verwendete Konzentration unterschiedlicher Antibiotika im Agar-Medium zur Transformationskontrolle:
Phleomycin 80 µg/ml; Carboxin 4 µg/ml; Hygromycin 400 µg/ml; ClonNAT 300 µg/ml.

### Erläuterungen zum Sequenzprotokoll

**SEQ ID NO. 1:** DNA-Sequenz des Primers lb1.
**SEQ ID NO. 2:** DNA-Sequenz des Primers lb2 mit Sfi I Schnittstelle.
**SEQ ID NO. 3:** DNA-Sequenz des Primers rb1.
**SEQ ID NO. 4:** DNA-Sequenz des Primers rb2 mit Sfi I Schnittstelle.
**SEQ ID NO. 5:** DNA-Sequenz des Primers KK-lb.
**SEQ ID NO. 6:** DNA-Sequenz des Primers KK-rb.
**SEQ ID NO. 7:** DNA-Sequenz des Primers test-lb.
**SEQ ID NO. 8:** DNA-Sequenz des Primers test-rb.
**SEQ ID NO. 9:** DNA-Sequenz der Sfi I-a Schnittstelle aus dem Plasmid pBS-hhn.
**SEQ ID NO. 10:** DNA-Sequenz der Sfi I-b Schnittstelle aus dem Plasmid pBS-hhn.
**SEQ ID NO. 11:** DNA-Sequenz des Primers hph-Nco/Bam.
**SEQ ID NO. 12:** DNA-Sequenz des Primers hph-STOP.
**SEQ ID NO. 13:** DNA-Sequenz des Primers 3-hph.
**SEQ ID NO. 14:** DNA-Sequenz des Primers 5-hsp.
**SEQ ID NO. 15:** DNA-Sequenz des Primers hsp-Nco.
**SEQ ID NO. 16:** DNA-Sequenz des Xho I/Sac I-Inserts (*hph*-Knockout-Kassette) aus dem Plasmid pBS-hhn.

### Erläuterungen zu den Abbildungen

### Abbildung 1: Schematische Darstellung des erfindungsgemäßen Verfahrens

A: Generierung der ein Gen (grauer Balken) flankierenden Bereiche (weiße Kästen) durch PCR mit den Außenprimern lb1/rb1 und den Innenprimern lb2/rb2. Die Primer lb2 und rb2 sind über die flankierenden Bereiche hinaus mit zwei unterschiedlichen Schnittstellen für ein Restriktionsenzym (hier: Sfi I) verlängert. (G = Guanosin, C = Cytosin, N = beliebige Base, abc = beliebige Abfolge dreier Basen; def= zu abc komplementäre Sequenz; uvw = beliebige, aber von abc verschiedene Abfolge dreier Basen; xyz= zu uvw komplementäre Sequenz).
B: Schneiden der Amplifikationsprodukte aus Schritt A mit einem Restriktionsenzym (hier: Sfi I), wodurch zwei nicht kompatible überhängende Enden entstehen.
C: Ligation einer Knockout-Kassette (schraffierter Balken), die zu den flankierenden Bereichen kompatible Schnittstellen desselben Restriktionsenzyms besitzt, zwischen die beiden flankierenden Bereiche des zu untersuchenden Gens.
D: Reamplifizierung des Ligationsproduktes aus Schritt C mit PCR unter Einsatz der Primer lb1 und rb1.
E: Transformation des Amplifikationsproduktes aus Schritt D in eine Rezipientenzelle.
F: Kontrolle der Transformation und der erfolgreichen homologen Rekombination durch Ganz-Zell-PCR mit den Primern test-lb/test-rb, die jeweils an Bereiche außerhalb der in Schritt A gewählten flankierenden Bereiche binden, in Kombination mit den Primern KK-1b und KK-rb, die jeweils an Bereiche innerhalb der Knockout-Kassette binden.

### Abbildung 2: Schematische Darstellung des Knockout-Konstruktes

- F (weißer Balken):: symbolisiert die beiden flankierenden Bereiche des interessierenden Gens.
- S (schwarzes Quadrat):: symbolisiert die beiden unterschiedlichen Restriktionsschnittstellen.
- M (schraffierter Balken):: symbolisiert das Marker-Gen einschließlich Promotor und Terminator.

### Abbildung 3: Schematische Darstellung der Fragmente zum Herstellen der Knockout-Kassette

A: Fragment A mit Marker-Gen (M) und Terminator (T), sowie den Restriktionsschnittstellen R1 bis R4 und RS-b.
B: Fragment B mit Promotor (P) und den Restriktionsschnittstellen R1, R5, R6 und RS-a.
C: Fragment C mit der DNA des Plasmidrückgrats (Plasmid) und den Restriktionsschnittstellen R4 und R5.

Weiße Balken in den drei Fragmenten deuten optionale kurze DNA-Zwischenräume unterschiedlicher Länge zwischen den einzelnen Elementen an. R1 bis R6 bezeichnen Restriktionsschnittstellen mit palindromischer DNA-Sequenz für sechs unterschiedliche Restriktionsenzyme. RS-a und RS-b bezeichnen zwei unterschiedliche Restriktionsschnittstellen mit nicht-palindromischer Sequenz für dasselbe Restriktionsenzym.

### Abbildung 4: Schematische Darstellung des Knockout-Konstruktes

Die verwendeten Abkürzungen sind mit denen aus Abb. 3 identisch.

### Abbildung 5: Physikalische Karte des Plasmids pBS-hhn.

Das Plasmid enthält den verkürzten *hsp70*-Promotor (hsp = heat shock protein) aus *U. maydis* und das *hph*-Gen (Hygromycin-Phosphotransferase-Gen) mit dem NOS-Terminator. Das Plasmid enthält weiterhin acht Schnittstellen für Restriktionsenzyme: Not I (bei Nucleotid 957, aus Nocardia otitidis-caviarum mit der Sequenz 5'-GC↓GGCCGC-3'), Nco I (bei Nucleotid 1634 und 1988 mit der Sequenz 5'-C↓CATGG-3'), Eco RI (bei Nucleotid 1743 mit der Sequenz 5'-G↓AATTC-3'), Xho I (bei Nucleotid 2555 mit der Sequenz 5'-C↓TCGAG-3'), Kpn I (bei Nucleotid 2570 mit der Sequenz 5'-GGTAC↓C-3'), sowie bei Nucleotid 659 die Schnittstelle Sfi I-b und bei Nucleotid 2543 die Schnittstelle Sfi I-a mit den im Sequenzprotokoll angegebenen Sequenzen (SEQ ID NO. 9 und SEQ ID NO. 10).

### Abbildung 6: Deletion des b-Kreuzungstyp-Locus

A: Gel-elektrophoretische Auftrennung der PCR-Produkte zur Amplifikation von LB und RB (60°C und 65°C Annealing-Temperatur) sowie des Sfi I geschnittenen Vektors pBS-hhn (LB = linke Flanke des b-Locus-Gens, RB = rechte Flanke des b-Locus-Gens).
B: Gel-elektrophoretische Auftrennung der mit Sfi I restringierten und Gel-gereinigten Flanken LB und RB sowie der *hph*-Kassette.
C: Gel-elektrophoretische Auftrennung der Ligationsprodukte.
D: Gel-elektrophoretische Auftrennung nach der Reamplifikation der Banden A und B (aus Abb. C) bzw. des gesamten Ligationsansatzes (C).
E: Kreuzungsanalyse von je 20 Transformanden aus Ansatz A und C gegen den Stamm FB2 (*a2b2*)
F: Southern-Analyse von je 6 Transformanden aus Ansatz A und C: Genomische DNA der Transformanden und des Wildtyp-Stamms FB2 wurde mit Sal I geschnitten, gel-elekrophoretisch aufgetrennt und in einer Southern-Blot Analyse mit dem *b*-Locus als Sonde hybridisiert. Bei Genaustausch entsteht statt einer 5.9 kb Bande eine 2.1 kb Bande.

### Abbildung 7: PCR-Analyse zur Kontrolle der Transformation

Gelelektrophoretische Auftrennung von PCR-Produkten mit den Primerkombinationen "KK-1b + lb1", "KK-1b + test-lb", "KK-rb + rb1" und "KK-rb + test-rb". Verwendet wurde die DNA von 10 verschiedenen Transformanden.
- Spur 1:: 1 kb Molekulargewichtsmarker (Größen in kb).
- Spuren 2 bis 6:: Entsprechen den in den Spuren 1 bis 5 in Abb. 6F untersuchten Transformanden.
- Spuren 7 bis 11:: Entsprechen den in den Spuren 7 bis 11 in Abb. 6F untersuchten Transformanden.
- Spur 12:: PCR-Reaktion ohne Zugabe von DNA.

### Literatur

Basse C.W., Stumpferl S., Kahmann R. (2000). Characterization of a Ustilago maydis gene specifically induced during the biotrophic phase: evidence for negative as well as positive regulation, *Mol. Cell. Biol.* 20, 329-339.

Bölker M., Bohnert H.U., Braun K.H., Gorl J., Kahmann R. (1995). Tagging pathogenicity genes in Ustilago maydis by restriction enzyme-mediated integration (REMI), *Mol. Gen. Genet.* 248, 547-552.

Fotheringham S., Holloman W.K. (1989). Cloning and disruption of Ustilago maydis genes, *Mol. Cell. Biol.* 9, 4052-4055.

Innis M.A., Gelfand D.H., Sninsky J.J., White T.J., eds. (1990). PCR Protocols: A Guide to Methods and Applications. (Academic Press, San Diego, USA).

Kronstad J.W., Wang J., Covert S.F., Holden D.W., McKnight G.L., Leong S.A. (1989). Isolation of metabolic genes and demonstration of gene disruption in the phytopathogenic fungus Ustilago maydis, *Gene* 79, 97-106.

Lehmler C., Steinberg G., Snetselaar K.M., Schliwa M., Kahmann R., Bölker M. (1997). Identification of a motor protein required forfilamentous growth in Ustilago maydis, *EMBO J.* 16, 3464-3473.

Quadbeck-Seeger C., Wanner G., Huber S., Kahmann R., Kämper J. (2000). A protein with similarity to the human retinoblastoma binding protein 2 acts specifically as a repressor for genes regulated by the b mating type locus in Ustilago maydis, *Mol. Microbiol.* 38, 154-166.

Regenfelder E., Spellig T., Andreas Hartmann A., Stephanie Lauenstein S., Bölker M., Kahmann R. (1997). G proteins in Ustilago maydis: transmission of multiple signals? *EMBO J*. 16, 1934-1942.

Spellig T., Bottin A., Kahmann R. (1996). Green fluorescent protein (GFP) as a new vital marker in the phytopathogenic fungus Ustilago maydis, *Mol. Gen. Genet.* 252, 503-509.

Tsukuda T., Carleton S., Fotheringham S., Holloman W.K. (1988). Isolation and characterization of an autonomously replicating sequence from Ustilago maydis, *Mol. Cell. Biol.* 8, 3703-3709.

Wang J., Holden D.W., Leong S.A. (1988). Gene transfer system for the phytopathogenic fungus Ustilago maydis, *Proc. Natl. Acad. Sci. USA* 85, 865-869.

## Patentansprüche

1. Verfahren zum Herstellen von Deletionsmutanten, **dadurch gekennzeichnet, dass** man
A) in einem ersten Verfahrensschritt die ein Gen flankierenden Bereiche durch PCR mit für diese Bereiche spezifischen Primern generiert, wobei über die beiden Innenprimer an den das Gen flankierenden Enden zwei unterschiedliche Schnittstellen für dasselbe Restriktionsenzym erzeugt werden,
B) in einem zweiten Verfahrensschritt die Amplifikationsprodukte mit dem für diese Schnittstellen spezifischen Restriktionsenzym schneidet, wodurch zwei nicht identische überhängende Enden entstehen,
C) in einem dritten Verfahrensschritt eine Knockout-Kassette, die die entsprechenden kompatiblen Schnittstellen besitzt, zwischen die beiden flankierenden Bereiche ligiert,
D) in einem vierten Verfahrensschritt das Ligationsprodukt mit PCR unter Einsatz der Außenprimer aus dem ersten Verfahrensschritt reamplifiziert und
E) in einem fünften Verfahrensschritt das Amplifikationsprodukt aus dem vierten Schritt in eine Rezipientenzelle transformiert.

2. Verfahren gemäß Anspruch 1, **dadurch gekennzeichnet, dass** man eukaryotische Zellen zum Herstellen von Deletionsmutanten einsetzt.

3. Verfahren gemäß Anspruch 2, **dadurch gekennzeichnet, dass** man Pilze einsetzt.

4. Verfahren gemäß Anspruch 3, **dadurch gekennzeichnet, dass** man Basidiomycota oder Ascomycota einsetzt.

5. Verfahren gemäß Anspruch 4, **dadurch gekennzeichnet, dass** man Ustilagomycetes oder Hemiascomycetes einsetzt.

6. Verfahren gemäß Anspruch 5, **dadurch gekennzeichnet, dass** man Ustilago maydis oder Saccharomyces cerevisiae einsetzt.

7. Verfahren gemäß Anspruch 6, **dadurch gekennzeichnet, dass** man Ustilago maydis-Zellen einsetzt.

8. Verfahren gemäß Anspruch 1, **dadurch gekennzeichnet, dass** die flankierenden Bereiche des zu deletierenden Gens eine Größe zwischen 60 bp und 2000 bp besitzen.

9. Verfahren gemäß Anspruch 8, **dadurch gekennzeichnet, dass** die flankierenden Bereiche des zu deletierenden Gens eine Größe zwischen 600 bp und 1500 bp besitzen.

10. Verfahren gemäß Anspruch 9, **dadurch gekennzeichnet, dass** die flankierenden Bereiche des zu deletierenden Gens eine Größe zwischen 800 bp und 1200 bp besitzen.

11. Verfahren gemäß Anspruch 1, **dadurch gekennzeichnet, dass** über die Innenprimer zwei unterschiedliche Schnittstellen für dasselbe Restriktionsenzym generiert werden, wobei das Restriktionsenzym eine definierte Erkennungssequenz von mindestens 6 bp besitzt, die durch eine variable Restriktionssequenz von mindestens 3 bp unterbrochen ist und vom Enzym so geschnitten wird, dass überhängende Enden entstehen.

12. Verfahren gemäß Anspruch 11, **dadurch gekennzeichnet, dass** das Restriktionsenzym eine definierte Erkennungssequenz von mindestens 8 bp besitzt, die durch eine variable Restriktionssequenz von mindestens 5 bp unterbrochen ist.

13. Verfahren gemäß Anspruch 12, **dadurch gekennzeichnet, dass** als Restriktionsenzym Sfi I verwendet wird.

14. Verfahren gemäß Anspruch 13, **dadurch gekennzeichnet, dass** die zwei unterschiedlichen Erkennungs- und Restriktionsschnittstellen die Nucleotid-Sequenzen Sfi I-a und Sfi I-b gemäß SEQ ID NO. 9 und SEQ ID NO. 10 besitzen.

15. Verfahren gemäß Anspruch 1, **dadurch gekennzeichnet, dass** die im dritten Verfahrensschritt benötigte Knockout-Kassette ein Marker-Gen trägt.

16. Verfahren gemäß Anspruch 15, **dadurch gekennzeichnet, dass** das Marker-Gen ein Resistenz-Gen gegen ein Antibiotikum, gegen ein Insektizid, gegen ein Herbizid oder gegen ein Fungizid ist.

17. Verfahren gemäß Anspruch 16, **dadurch gekennzeichnet, dass** das Marker-Gen ein Resistenz-Gen gegen ein Antibiotikum ausgewählt aus der Reihe Hygromycin, Carboxin und Phleomycin ist.

18. Verfahren gemäß Anspruch 17, **dadurch gekennzeichnet, dass** das Marker-Gen das Hygromycin-Phosphotransferase-Gen ist.

19. Verfahren gemäß Anspruch 1, **dadurch gekennzeichnet, dass** man zur Transformation Protoplasten verwendet.

20. Verfahren zum Auffinden essentieller Gene, **dadurch gekennzeichnet, dass** man die in Anspruch 1 genannten Verfahrensschritte (A) bis (E) ausführt und anschließend den Wachstumsphänotyp bestimmt.

21. Verfahren gemäß Anspruch 20, **dadurch gekennzeichnet, dass** man den Wachstumsphänotyp bestimmt, indem man den untersuchten Organismus oder Zellen von diesem einerseits auf Normalmedium (Test 1) und andererseits auf Selektionsmedium (Test 2) wachsen lässt und die Ergebnisse im Wachstumsverhalten vergleicht.

22. Verfahren zum Identifizieren von Targets für die Wirkstoffsuche, **dadurch gekennzeichnet ist, dass** man ein essentielles Gen in einem Verfahren gemäß Anspruch 20 identifiziert und anschließend Ansätze entwickelt, die Expression des Gens zu verhindern oder die Funktion des Genproduktes zu inhibieren.

23. Verfahren gemäß Anspruch 1, **dadurch gekennzeichnet, dass** man Deletionsmutanten im Hochdurchsatz herstellt.

24. Verwendung eines Verfahrens gemäß Anspruch 1 zum Herstellen von Deletionsmutanten im Hochdurchsatz.

25. Knockout-Kassette enthaltend eine erste nicht-palindromische Restriktionsschnittstelle, ein Marker-Gen mit Promotor und Terminator, sowie eine zweiten nicht-palindromische Restriktionsschnittstelle und gegebenenfalls weitere palindromische Restriktionsschnittstellen.

26. Knockout-Kassette gemäß Anspruch 24, **dadurch gekennzeichnet, dass** die nicht-palindromischen Restriktionsschnittstellen die Nucleotidsequenzen gemäß SEQ ID NO. 9 und SEQ ID NO. 10 besitzen.

27. Knockout-Kassette gemäß Anspruch 24, **dadurch gekennzeichnet, dass** das Hygromycin-Phosphotransferase-Gen als Marker-Gen enthalten ist.

28. Knockout-Kassette gemäß Anspruch 24, **dadurch gekennzeichnet, dass** der *hsp70*-Promotor aus *U. maydis* enthalten ist.

29. Knockout-Kassette gemäß Anspruch 24, **dadurch gekennzeichnet, dass** der Agrobakterium NOS-Terminator enthalten ist.

30. Knockout-Kassette gemäß einem der Ansprüche 24 bis 28, **dadurch gekennzeichnet, dass** die Knockout-Kassette die Nucleotid-Sequenz gemäß SEQ ID NO. 16 besitzt.

31. Verwendung einer Knockout-Kassette gemäß einem der Ansprüche 24 bis 29 im Verfahren zum Herstellen von Deletionsmutanten.

32. Verfahren zum Herstellen einer Knockout-Kassette, **dadurch gekennzeichnet, dass** man
a) in einem ersten Schritt aus einem Plasmid I mit den Primern 1 und 2 ein Marker-Gen amplifiziert, wodurch an beiden Enden des Marker-Gens unterschiedliche Restriktionsschnittstellen R1 und R2 eingeführt werden, und das PCR-Produkt anschließend mit den Enzymen für diese Schnittstellen R1 und R2 restringiert, und
b) in einem folgenden Schritt das so erhaltene PCR-Produkt mit einem Terminator verknüpft, indem man ein Plasmid II mit den Enzymen für die Schnittstellen R1 und R2 restringiert und das durch die Restriktion freigesetzte Fragment gegen das PCR-Fragment mit dem Marker-Gen aus Schritt (a) austauscht, wodurch man ein Plasmid III erhält, und
c) ausgehend von diesem Plasmid III in einer zweiten PCR mit den Primern 1 und 4 das Marker-Gen zusammen mit dem Terminator amplifiziert, wodurch am 5'-Ende wenigstens die Schnittstelle R1 und am 3'-Ende die Schnittstelle RS-b gefolgt von einer Schnittstelle R4 generiert werden, und das so erhaltene Fragment A anschließend in ein Plasmid IV kloniert, wodurch man Plasmid V erhält, und
d) ausgehend von Plasmid I in einer dritten PCR durch die Primer 5 und 6 eine funktionsfähige Promotorsequenz für das Marker-Gen amplifiziert, wodurch am 5'-Ende der Promotor-Sequenz die Schnittstellen R5 und RS-a und am 3'-Ende wenigstens die Schnittstelle R1 generiert werden, und das so erhaltene Fragment B anschließend in ein Plasmid IV kloniert, wodurch man das Plasmid VI erhält, und
e) in einem letzten Schritt i) das Fragment A durch Restriktion des Plasmids V mit den Enzymen R1 und R4 freisetzt, ii) das Fragment B durch Restriktion des Plasmids VI mit den Enzymen R5 und R1 freisetzt, iii) das Fragment C durch Restriktion eines Plasmids VII mit den Enzymen R5 und R4 freisetzt und iv) die drei erhaltenen Fragmente zusammen ligiert, wodurch man ein Plasmid VIII erhält, und
f) das Plasmid VIII aus Schritt (e) mit dem Restriktionsenzym für die Schnittstellen RS-a und RS-b schneidet.
